**Europäisches Patentamt**

**European Patent Office**

**Office européen des brevets**

(11) Veröffentlichungsnummer : **0 336 236 B1**

(19)

# (12) EUROPÄISCHE PATENTSCHRIFT

(45) Veröffentlichungstag der Patentschrift :
**14.10.92 Patentblatt 92/42**

(51) Int. Cl.⁵ : **A61K 7/06, A23K 1/175**

(21) Anmeldenummer : **89105267.2**

(22) Anmeldetag : **23.03.89**

(54) **Mittel und Verfahren zur qualitativen und quantitativen Förderung des Haarwuchses bei Mensch und Nutztier.**

(30) Priorität : **28.03.88 DD 314108**

(43) Veröffentlichungstag der Anmeldung :
**11.10.89 Patentblatt 89/41**

(45) Bekanntmachung des Hinweises auf die
Patenterteilung :
**14.10.92 Patentblatt 92/42**

(84) Benannte Vertragsstaaten :
**AT BE CH DE ES FR GB GR IT LI LU NL SE**

(56) Entgegenhaltungen :
**CH-A- 653 251**
**DE-A- 2 307 596**

(73) Patentinhaber : **Weuffen, Wolfgang,**
**Prof.Dr.sc.med.Dr.rer.nat.**
**Ringstrasse 39**
**O-2201 Guest (DE)**
Patentinhaber : **Kramer, Axel, Prof.Dr.sc.med.**
**Georg-Engel-Strasse 20**
**O-2200 Greifswald (DE)**
Patentinhaber : **Hiepe, Theodor, Prof.Dr.sc.med.vet.**
**Dr.h.c.**
**Harnischweg 9**
**O-1170 Berlin (DE)**
Patentinhaber : **Koch, Stephan, Dr.med.**
**Schulstrasse 19**
**O-1242 Bad Saarow (DE)**
Patentinhaber : **Meffert, Hans, Prof.Dr.sc.med.**
**Oranienburger Strasse 89**
**O-1020 Berlin (DE)**
Patentinhaber : **Minnich, Siegfried**
**Friedrich-Löffler-Strasse 2**
**O-2200 Greifswald (DE)**
Patentinhaber : **Thürkow, Bodo, Dr.rer.nat.,**
**Dipl.-Chem.**
**Meisengrund 14**
**O-2030 Demmin (DE)**
Patentinhaber : **Völzke, Marietta**
**Lübecker Strasse, Ausbau 4**
**O-2120 Ueckermünde (DE)**

Patentinhaber : **Völzke, Norbert**
**Lübecker Strasse, Ausbau 4**
**O-2120 Ueckermünde (DE)**
Patentinhaber : **Winetzka, Hans**
**Neuer Weg 1**
**O-2201 Mesekenhagen (DE)**

(72) Erfinder : **Weuffen, Wolfgang,**
**Prof.Dr.sc.med.Dr.rer.nat.**
**Ringstrasse 39**
**O-2201 Guest (DE)**
Erfinder : **Kramer, Axel, Prof.Dr.sc.med.**
**Georg-Engel-Strasse 20**
**O-2200 Greifswald (DE)**
Erfinder : **Hiepe, Theodor, Prof.Dr.sc.med.vet.**
**Dr.h.c.**
**Harnischweg 9**
**O-1170 Berlin (DE)**
Erfinder : **Koch, Stephan, Dr.med.**
**Schulstrasse 19**
**O-1242 Bad Saarow (DE)**
Erfinder : **Meffert, Hans, Prof.Dr.sc.med.**
**Oranienburger Strasse 89**
**O-1020 Berlin (DE)**
Erfinder : **Minnich, Siegfried**
**Friedrich-Löffler-Strasse 2**
**O-2200 Greifswald (DE)**
Erfinder : **Thürkow, Bodo, Dr.rer.nat., Dipl.-Chem.**
**Meisengrund 14**
**O-2030 Demmin (DE)**
Erfinder : **Völzke, Marietta**
**Lübecker Strasse, Ausbau 4**
**O-2120 Ueckermünde (DE)**
Erfinder : **Völzke, Norbert**
**Lübecker Strasse, Ausbau 4**
**O-2120 Ueckermünde (DE)**
Erfinder : **Winetzka, Hans**
**Neuer Weg 1**
**O-2201 Mesekenhagen (DE)**

(74) Vertreter : **Jönsson, Hans-Peter et al**
**Patentanwälte von Kreisler Selting Werner**
**Deichmannhaus am Hauptbahnhof**
**W-5000 Köln 1 (DE)**

EP 0 336 236 B1

## Beschreibung

Die Erfindung betrifft ein Mittel und ein Verfahren zur Erhaltung bzw. Förderung des Haarwuchses beim Menschen sowie ein Mittel und ein Verfahren zur Qualitätsverbesserung und Ertragssteigerung bei der Bildung von Haar, Wolle, Fell, Pelz und Federn beim Nutztier.

Allgemein ist bekannt, daß krankheitsbedingte Schädigungen und auch im täglichen Leben übliche Manipulationen wie Haarwaschen, -bürsten, -fönen, -färben und -verformen zu vorzeitigem Haarausfall und zur Hemmung des Haarwachstums führen können. Derartig hervorgerufene oberflächliche Substanzverluste am Haar versucht der Mensch durch Haarpackungen und Haarfestiger auszugleichen. Die geschädigte Kopfhaut wird mit Kopfhautpackungen und -salben behandelt. Oft werden auch prophylaktische Behandlungsmaßnahmen wie Haartinkturen und Kurpackungen für die Kopfhaut und Haare angewandt, die jedoch auf keinem konkreten Wirkmechanismus beruhen, sondern nur das Wohlbefinden des Behandelten steigern.

Für die Haarpflege bzw. die Förderung des Haarwachstums kommt es jedoch darauf an, über ein Mittel und ein Verfahren zu verfügen, das bereits beim ungeschädigten Haar zu einer positiven Beeinflussung im Sinne einer Qualitätsverbesserung und Haarwuchsförderung führt.

Die bisher hierfür bekannten Mittel haben sich in der Praxis nicht durchsetzen können. Einerseits sind sie in ihrer Anwendung nicht geeignet, nachweislich das Haarwachstum auf physiologischem Weg zu stimulieren, andererseits waren die erwünschten Effekte klinisch nicht reproduzierbar oder sogar mit unerwünschten Nebenwirkungen verbunden. Hinzu kommt, daß für die bisher unter suchten Wirkstoffe ausreichende Vorstellungen zum Wirkungsmechanismus fehlen, was die Vielzahl vorgeschlagener chemischer Strukturen bzw. natürlicher Extrakte erklärt.

Das betrifft die unterschiedlichsten pflanzlichen Extrakte, Öle bzw. Zubereitungen, tierische Extrakte, Amnionflüssigkeiten und Hormone, gefäßerweiternde und hautreizende Wirkstoffe wie Iso-thiocyanate, Alkohole, Äther, Phenolderivate oder Kohlendioxid, natürliche Fettsäuren, Schwefel und Thiosulfate, Gibberelline, Elastase bzw. Kollagenextrakte und mikrobielle Extrakte.

Bei Zubereitungen aus mikrobiellen und pflanzlichen Extrakten, in geringerem Maße auch bei Zubereitungen tierischer Herkunft ist davon auszugehen, daß diese je nach dem natürlichen Gehalt der Mikroorganismen, Pflanzen bzw. tierischen Gewebe in geringen Mengen auch Thiocyanat ($SCN^-$) im Bereich von Mikrogramm bis zu einigen Milligramm $SCN^-$/l enthalten. Das trifft auf alle Haarwuchs- und Haarpflegemittel zu, in denen biologische Rohstoffe eingesetzt sind. So enthalten z. B. Shampoos mit Eikonzentrat 0,2 mg - 1,3 mg $SCN^-$/l (eigene Befunde, unveröffentlicht).

In der DE-PS 303 928.1 ist ein Haarwuchsmittel auf der Grundlage eines Meerrettichextraktes beansprucht, das höchstens 28 mg $SCN^-$/l enthalten soll. In den Unteransprüchen 5 und 6 dieser Patentschrift wird die Aussage gemacht, daß das Haarwuchsmittel gegebenenfalls $Cu^{2+}$ und $SCN^-$ in Mengen von 1,5 - 6 mg/l bzw. 1 - 5 mg/l enthält. In der Erfindungsbeschreibung werden die Haarwuchseffekte auf Allylisothiocyanat, Majoranextrakt und Wachholderbeerenextrakt zurückgeführt, wodurch die Keratinbildung verbessert, der unangenehme Geruch des Meerettichextraktes überdeckt und die Klebrigkeit beseitigt sowie die Lagerfähigkeit günstig beeinflußt wird. Bei krankheitsbedingtem Haarausfall kann das Haarwachstum durch erfolgreiche Behandlung der zugrundeliegenden Krankheit gesteigert werden. Es ist seit langem bekannt, daß durch Nebennierenrindenhormone, Geschlechtshormone oder deren Antagonisten das Haarwachstum vorübergehend gesteigert werden kann. Wegen kaum vermeidbarer schwerer Nebenwirkungen werden solche Behandlungen nur noch selten und nur unter strenger ärztlicher Kontrolle durchgeführt. Hierbei handelt es sich jedoch ausschließlich um Wirkstoffanwendungen in Form von Arzneimitteln, so daß eine Anwendung als Haarwuchs- und Haarpflegemittel, Kosmetikum bzw. Nutritivum (d.h. als Futterzusatzstoff zur Verbesserung bestimmter Leistungsparameter für Nutztiere) nicht in Frage kommt.

Neuerdings wurde in einigen Ländern die Zulassung des Wirkstoffs Minoxidil, welches in den PCT-Patentschriften WO 83/02558 und WO 87/00427 sowie in den japanischen Offenlegungsschriften 61/165311 und 61/165312 genannt ist, zur äußerlichen Stimulierung des Haarwachstums beantragt. Nach zweimal täglicher Anwendung einer Minoxidil-Lösung hatte sich innerhalb eines Jahres bei etwa 1/3 aller Probanden mit Haarausfall vom männlichen Typ (androgener Haarausfall) das Haarwachstum kosmetisch akzeptabel gebessert. Da bei Anwendung eines Pharmakons wie Minoxidil das Haarwachstum nicht auf physiologischem Wege angeregt wird, sind Nebenwirkungen zu erwarten, die sich zunächst in einer Blutdrucksenkung und einer Erhöhung der Herzschlagfrequenz äußerten (Olsen, E. A.; M. S. Weiner, E. R. Delong und S. R. Tinell: "Topical Minoxidil in early male pattern baldness". Journal of American Academic. Dermatol. 13 (1985) pages 185-192). Auch hier trifft zu, daß eine Anwendung als Pflegemittel nicht in Frage kommt.

Hinzu kommen je nach eingesetzten Mitteln bzw. Wirkstoffzubereitungen weitere Nachteile wie die Möglichkeit mikrotoxischer Langzeitwirkungen bei ständiger langjähriger Anwendung, unangenehme Beeinflussung des Haares wie Verkleben und unangenehmer Geruch, lokale Reizung, erhöhte Empfindlichkeit gegen UV-Strahlung, so daß nur eine kurzzeitige Anwendung im Winterhalbjahr möglich ist, verschlechterte Eigenschaften des Haares wie schlechtere Kämmbarkeit, verminderte Elastizität, herabgesetzter Glanz, erhöhte Sprödigkeit und Sensibilität, verminderte Widerstandsfähigkeit sowie Adaption an den Wirkstoff, was nur dann nicht zu erwarten ist, wenn ein physiologischer Mechanismus auf physiologische Art und Weise stimuliert wird.

Beim gesunden Nutztier wird als primär wirksame Einflußgröße auf das Haarwachstum bzw. für die Erhaltung der natürlichen Beschaffenheit und Funktion des Haares eine optimale Ernährung angesehen, um eine hohe Qualität bei Wolle, Pelz, Fell, Leder und Federn zu erzielen. Bei Verfütterung von eiweißhaltigen Futtermitteln wird z. B. die Wollsynthese und Lebendmasse beim Schaf gesteigert. Es entsteht ein dichtes, weiches und glänzendes, Vlies. Eine Erhöhung des Kohlenhydratgehaltes von Futtermitteln führt zum Anstieg von Thiol-Gruppen (SH-Gruppen) in der Haut, woraus ebenfalls eine Förderung des Haarwachstums resultiert. Gleichzeitig werden die Struktur und physikalischen Eigenschaften der Haare verbessert. Nach Doehner, H., Handbuch der Schafzucht und Schafhaltung, Band I, Parey-Verlag Berlin Hamburg, 1954, sowie Gebhard, G., Tierproduktion - Tierernährung, VEB Deutscher Landwirtschaftsverlag Berlin, 1981 und Schwark, H. J.; Jankowski, St.; Veress, L.; Internationales Handbuch der Tierproduktion - Schafe; VEB Deutscher Landwirtschaftsverlag Berlin, 1981 bestehen weitere Möglichkeiten zur Förderung des Haarwachstums in einer zusätzlichen Verabreichung von Schwefel und schwefelhaltigen Aminosäuren, insbesondere von Cystin, aber auch von Methionin.

Ähnliche Wirkungen sind von Calcium-, Natrium- und Ammoniumsulfat bekannt. Allerdings beschreibt Kiok, P. in: "Der Einfluß der Ernährung auf die Qualität des Wollertrages bei Merino-fleischschafen unter besonderer Berücksichtigung der Beifütterung von schwefelhaltigen Aminosäuren und reinem Schwefel in eiweißreichem Kraftfutter", Dissertation, Veterinärmedizinische Fakultät der Humboldt-Universität zu Berlin, 1969, daß bei optimaler alimentärer, das heißt über die Nahrungsaufnahme stattfindender Schwefelversorgung keine stimulierenden Effekte auf Wachstum und Qualität des Haarkleides feststellbar sind.

Eine andere Möglichkeit der Steigerung des Haarwachstums ist auf hormonellem Weg gegeben. Allerdings ist hiermit langfristig keine Verbesserung erreichbar. Ein weiterer Nachteil ist die Restkontamination des Schlachtfleisches mit Hormonresten.

Die verschiedenen Möglichkeiten der Optimierung der Tierernährung haben alle den Nachteil, daß es sich um ökonomisch aufwendige Maßnahmen handelt, die zudem schwer steuerbar sind, da sie jeweils von dem aktuellen Futterangebot und der Zusammensetzung der Futterinhaltsstoffe abhängig sind.

Der Erfindung liegt die Aufgabe zugrunde, ein Haarkosmetikum für den Menschen bzw. ein physiologisches Haarwuchsmittel für Mensch und Nutztier zu schaffen, das auf Grund seiner physikochemischen und biochemischen Eigenschaften in der Lage ist, das quantitative Haarwachstum und die Qualität der Haarbildung zu verbessern, ohne daß damit eine toxische Belastung bzw. Gefährdung für den Organismus oder eine Umweltbelastung entsteht. Gleichzeitig soll eine erhöhte Widerstandsfähigkeit von Haar, Wolle, Fell, Pelz, Leder bzw. Federn gegenüber Umwelteinflüssen und Beanspruchungen im gewerblichen Verarbeitungsprozeß erreicht werden.

Die Erfindung beruht auf der überraschenden Erkenntnis der Wirkung von einem oder mehreren Salzen der Thiocyansäure als alleiniger und nichttoxischer Wirkstoff für eine qualitative und quantitative Förderung des Haarwuchses bei Mensch und Nutztier.

Die bekannten Lösungen des Standes der Technik, wonach auch geringe Mengen von Thiocyanaten ($SCN^-$) im Zusammenwirken mit anderen Wirkstoffen eingesetzt werden, gaben für diese Erkenntnis des Thiocyanateffektes keine Anregung. Vielmehr wurde, z. B. in der DE-PS 30 39 281 die Feststellung getroffen, daß der, durch den Einsatz pflanzlicher Rohstoffe bedingte, offensichtlich schädliche Einfluß von $SCN^-$ nur durch Zusatz geeigneter kompensierender Mittel wie z. B. Kupfer(II)-Ionen auszugleichen ist. Darüber hinaus ist dieser bekannten Lösung entnehmbar, daß wegen der offenbar toxischen Wirkung die einzusetzenden Mengen von $SCN^-$ weitgehendst zu minimieren sind. Aus toxikologischen Gründen wurde eine Gehaltsbegrenzung für $Cu^{2+}$ auf 1,5 bis 6 mg/l und für $SCN^-$ auf 1 bis 5 mg/l vorgesehen.

Durch die Erfinder wurde das in der DE-PS vorgeschlagene Gemisch, bestehend aus 1 Teil $SCN^-$ und 6 Teilen $Cu^{2+}$ auf Stabilität geprüft. Im Versuch wurde dazu folgender Ansatz gewählt: 160 mg $Cu^{2+}$/l (hergestellt aus $CuCO_4 \cdot 5 H_2O$) und 28 mg $SCN^-$/l (hergestellt aus KSCN). Der $SCN^-$-Gehalt wurde mit der Chlorcyan-Pyridin-Barbituratsäure-Methode ermittelt (Methode nach Weuffen und Below: "Zur Anwendbarkeit der Chlorcyan-Pyridin-Barbituratsäurebestimmung für die Thiocyanatbestimmung in Serum und Urin". Wissenschaftliche Zeitschrift der Ernst-Moritz-Arndt-Universität Greifswald, Medizinische Reihe 36 (1987) Seiten 120-124, ISSN 0138-1067).

Überraschenderweise fiel innerhalb von 25 Tagen nach Herstellung dieses Gemisches der $SCN^-$-Gehalt von 28 mg $SCN^-$/l auf 19,3 mg $SCN^-$/l, das heißt um 31 % ab. Bereits eine Stunde nach Herstellung des Gemisches war eine Reduzierung des $SCN^-$-Gehaltes um 10 % feststellbar. In einer Vergleichslösung aus 28 mg $SCN^-$/l (hergestellt aus KSCN) blieb demgegenüber der $SCN^-$-Gehalt erwartungsgemäß konstant. Die Ursachen für die Abnahme der SCN⁻-Konzentration sind in der Bildung von sehr schwer wasserlöslichem CuSCN zu sehen, weil in Abhängigkeit vom Redoxpotential aus $Cu^{2+}$ das $Cu^{1+}$ gebildet werden kann, das mit SCN⁻ zu CuSCN reagiert. In Abhängigkeit von äußeren Einflußfaktoren auf das Redoxpotential (z. B. atmosphärischer Sauerstoff, Staub) nimmt der SCN⁻-Gehalt ungleichmäßig, aber insgesamt fortwährend ab. Unter diesen Gesichtspunkten handelt es sich bei dem, in der DE-PS 3039281 beanspruchten Gehalt an $Cu^{2+}$ und SCN⁻ nicht um einen langzeitstabilen, biologisch aktiven und gewünschten Zusatz. Gleichzeitig steht aber durch den raschen Verlust an freien Thiocyanationen nur noch ein geringerer Anteil von SCN⁻ zur Verfügung.

Das erfindungsgemäße neuartige Mittel ist als Pflege mittel, als Bestandteil in Kosmetika oder als Futter zusatzstoff für folgende Zielstellungen anwendbar:

- Unterstützung des physiologischen Haarwuchses beim Menschen:

Zur Gewährleistung einer gesunden Haarbildung ist eine regelmäßige externe Anwendung von Kindheit an als Zusatz zu Haarwaschmitteln zweckmäßig. Hierfür kommt ein Konzentrationsbereich von 5 bis 10 mg Thiocyanat/l Haarwaschmittel (0,0005 % - 0,001 %) in Frage.

- Verhütung von Haarschäden und nachfolgendem Haarverlust beim Menschen:

Hierbei geht es vor allem um die Herabsetzung haarschädigender Einflüsse, z. B. durch Dauerwelle, Haarfärbung u. ä..
Vorteilhaft erfolgt die externe Anwendung in Form von Einreibungen nach der Haarwäsche mit Haarwässern auf alkoholischer Grundlage, ebenso als Zusatz zu Shampoos, Kosmetika bzw. Haarpflegemitteln. In diesen Anwendungsformen ist vorzugsweise ein Konzentrationsbereich von 10 mg - 1000 mg SCN⁻ /l (0,001 % - 0,1 %) zu wählen.
Zur Vorbeugung eines Haarausfalls ist es weiterhin zweckmäßig, die lokale Applikation des Thiocyanats mit vegetabilen thiocyanatreichen Rohstoffen durchzuführen und gleichzeitig eine entsprechende alimentäre thiocyanatreiche Kostform zu gewährleisten.

- Förderung der Haarneubildung bei toxischer Belastung des Menschen:

Bei toxisch beeinträchtigtem Haarwachstum, z. B. verursacht durch Zytostatika oder Strahlentherapie, ist durch externe und/oder interne Thiocyanatanwendung ein wachstumserhaltender und -fördernder Effekt erreichbar, solange die Haarwurzeln nicht irreversibel geschädigt bzw. atrophiert sind. Bei eingetretenem androgenen Haarausfall ist daher kein Effekt zu erwarten, wohl aber bei rechtzeitiger prophylaktischer Anwendung. Extern (oral) bzw. intern (alimentär) wird das Haarwuchsmittel in für den Menschen physiologisch angepaßten Dosierungen angewendet. Für den Menschen kommt die orale Gabe nur bei besonderer Indikation in Frage, z. B. bei positiver Beeinflussung einer regelwidrigen Haarbildung innerhalb eines zeitlich limitierten Anwendungszeitraums. Hierbei kommt als Dosisbereich für die initiale Gabe etwa 50 mg/d SCN⁻, als tägliche Erhaltungsdosis etwa 20 mg/d, in Frage. Dabei hängt die Gesamtanwendungsdauer von den individuellen Voraussetzungen des einzelnen Menschen ebenso wie von anderen Faktoren ab, wie z. B. eine vorausgegangene toxische Belastung mit anschließendem Haarausfall. Eine intervallmäßige Anwendung von ca. 10 - 20 Tagen Applikation und etwa 10 - 30 Tagen und mehr applikationsfreiem Intervall stellt lediglich eine Variante dar. Die anzuwendenden Dosisbereiche liegen etwa vierbis achtfach über der SCN⁻-Aufnahme, wie sie bei durchschnittlichen Ernährungsformen erreicht wird.

- Unterstützung des physiologischen Haarwuchses beim Nutztier durch nutritive Anwendung als Futterzusatzstoff:

Die Anwendung des erfindungsgemäßen Mittels beim Nutztier erfolgt vorzugsweise als Futterzusatzstoff (Nutritivum) durch Zumischung zum Futter oder durch Herstellung von mit dem Mittel angereicherten Futter, z. B. durch Pelletierung.
Diese Anwendungsform ist besonders zweckmäßig, weil es sich bei dem erfindungsgemäßen Mittel um einen natürlichen alimentären Inhaltsstoff des Futters handelt, der speziell unter industriemäßigen Haltungsbedingungen, aber auch bei Stall- und Käfighaltung nicht ausreichend mit der Nahrung zugeführt wird. Die Anwendungsdosierung liegt je nach der Tierspecies im Bereich von 0,5 mg bis 46 mg Thiocyanat/Kilogramm Körpermasse (kg KM). In Anpassung an die Ernährungsweise der jeweiligen Tierspecies wird die Dosis entweder kontinuierlich, z. B. bei Pelztieren, Federvieh, usw. oder diskontinuierlich, z. B. 1 - 3mal pro Woche, z. B. beim Schaf, verabreicht. Die Applikationsdauer ist von den Haltungsbedingungen abhängig. Bei Schafen empfiehlt sich die Gabe nach der 1. Schur und/oder im Winterhalbjahr über eine Dauer von 30 - 50 Tagen bzw. 4 - 5 Monaten. Beim Pelztier ist die Zufuhr vorteilhaft, sobald die Jungtiere von der Mutter abgesetzt sind. Nach 4 - 8wöchiger Gabe empfiehlt sich die Zwischenschaltung eines applikationsfreien Intervalls von etwa 4 Wochen. Danach wird Thiocyanat in Zyklen von etwa jeweils 4 Wochen mit etwa 2wöchigen applikationsfreien Intervallen bis zum Pelzen verabreicht.

In Abhängigkeit von der jeweiligen Tierspecies können verschiedene Salze der Thiocyansäure oder auch Salzgemische zur Anwendung kommen. So ist in Anlehnung an die natürliche Ernährungsweise z. B. bei pflanzenfressenden Tieren (Beispiel: Schaf) vorzugsweise ein Gemisch aus Kaliumthiocyanat und Natriumthiocyanat zu verwenden, bei dem der Anteil von Kaliumthiocyanat überwiegt. Bei fleischfressenden Nutztieren (Beispiel: Nerz) kann vorteilhaft ein Gemisch aus Kaliumthiocyanat und Natriumthiocyanat verwendet werden, bei dem der Anteil von Natriumthiocyanat überwiegt.

Durch eine nutritive Anwendung als Futterzusatzstoff werden folgende Effekte erzielt:

-- Steigerung der physiologischen Haarbildung bei hierfür in Frage kommenden Nutztierspecies, z. B. der Wollproduktion beim Schaf,

-- Verbesserung der Qualität der Haarbildung (Zunahme von Haardichte, Glanz, Festigkeit u. a.; damit Verbesserung der Qualität von Wolle, Pelzen, Leder und Federn),

-- Verbesserung des Gesundheitszustandes der Tiere (höhere Körpermassezunahme, bessere Aufzuchtleistung u. ä.).

- Unterstützung des physiologischen Haarwuchses beim Nutztier durch externe Anwendung:

Speziell bei Pelztieren ist die äußerliche Anwendung des erfindungsgemäßen Mittels als Ganzkörperbad eine zweckmäßige Form.

Erfindungsgemäß wurde gefunden, daß durch Salze der Thiocyansäure im für Mensch bzw. Nutztier physiologischen Konzentrationsbereich in einer jeweils der Biologie der Haarbildung und den Ursachen sowie dem Ausmaß einer Haarbildungsbeeinträchtigung angepaßten Form (Dosierung, Applikation, Zeitdauer, applikationsfreie Intervalle) bei lokaler und/oder oraler bzw. alimentärer Anwendung als Futterzusatzstoff die Haarbildung gefördert und die Qualität der Haarbildung verbessert wird. Beim Nutztier werden insbesondere die Wachstumsgeschwindigkeit des Haares bzw. Federkleides, die Haarmasse (z. B. Wolle) und die Haardichte im allgemeinen in Größenordnungen von 5 - 30 % im Vergleich zu bei analogen Bedingungen gehaltenen Kontrolltieren ohne zusätzliche Wirkstoffzufuhr gefördert. Das wirkt sich zugleich vorteilhaft auf die Eigenschaften von Haar, Wolle, Fell, Pelz, Leder bzw. Federn aus (verbesserter Glanz, verbesserte Fellstruktur, Haarstärke und -elastizität, Dichte und Gleichmäßigkeit des Haarkleides, Weichheit) sowie erhöhte Festigkeit im Verarbeitungsprozeß bzw. erhöhte Widerstandsfähigkeit gegenüber Umwelteinflüssen.

Durch die Anwendung des erfindungsgemäßen physiologischen Wirkstoffs sind bei physiologisch angepaßter Dosierung keine Nebenwirkungen zu erwarten und auch nicht beobachtet worden.

Darin besteht der entscheidende Unterschied des aufgefundenen Pflegemittels im Vergleich zu pharmakologischen Wirkstoffen. Da es sich um einen definierten Einzelwirkstoff handelt, ist eine exakte Dosierbarkeit gewährleistet.

Ausführungsbeispiele

Beispiel 1

Eine langjährige Anwendung über 15 Jahre bei männlichen erwachsenen Probanden in Form von einmal wöchentlichen Ganzkörperbädern mit mehrmaligem Eintauchen der Haare während des Badens führte zu einem dichten Haarwuchs mit festen Haaren und zu einer auffällig geringeren Empfindlichkeit gegenüber mechanischen Reizen (z. B. beim Ziehen an den Haaren). Wird ein längeres Intervall (z. B. drei oder mehr Monate) zwischen dieser Form der Thiocyanatanwendung eingelegt, wird die normale Schmerzempfindlichkeit der Haare auf mechanische Zugbeanspruchung wieder deutlich. Das Ergrauen der Haare wird offensichtlich nicht beeinflußt. Die Dosis betrug in den ersten fünf Jahren der Anwendung etwa 3 mg NaSCN/l Badewasser. Danach wurde sie auf 30 - 60 mg NaSCN/l Badewasser erhöht. Diese Erhöhung wirkte sich positiv auf die Elastizität (Frisierbarkeit) und Widerstandsfähigkeit des Haares gegenüber mechanischer Beanspruchung (Schmerzempfindlichkeit) aus.

Unerwünschte Nebenwirkungen waren nicht feststellbar, vielmehr war das Allgemeinbefinden subjektiv deutlich verbessert und zwar bei den höheren Dosierungen mit höherem subjektiven Effekt. Bei wiederholten Kontrollen des $SCN^-$-Serumspiegels war keine Beeinflussung durch die $SCN^-$-Anwendung als Ganzkörperbad feststellbar (durchschnittlicher $SCN^-$-Serumspiegel 2,2 ± 0,3 mg/l).

5

Beispiel 2

Bei toxischem Haarausfall, verursacht durch Kontakt mit einem algiciden und molluscuciden Farbanstrich, mit einem großflächigen Verlust von mehr als 2/3 der Kopfbehaarung, wurden gemäß Beispiel 1 mit einer Dosierung von 30 mg NaSCN/l Badewasser wöchentlich Ganzkörperbäder mit gleichzeitig mehrmaligem Eintauchen der Haare durchgeführt. Im Wechsel kamen Kopfbäder hinzu. Das geschah in den ersten 3 Wochen täglich, dann zweimal pro Woche, insgesamt 15 Monate lang. Schon nach etwa 8 - 14 Tagen setzte eine Wiederbehaarung mit der ursprünglichen Haarfarbe ein.

Beispiel 3

Bei 20 freiwilligen Probanden mit toxischem Haarausfall als Folge einer Cytostatikatherapie konnte durch täglich zweimalige Unterarmbäder mit einer Dosierung von 300 mg NaSCN/l Wasser die Haarbildung im Applikationsbereich günstig beeinflußt werden (ein Unterarm diente als Kontrolle, also nur Wasseranwendung, am anderen Unterarm wurde das erfindungsgemäße Mittel angewandt).

Beispiel 4

Fünf freiwillige weibliche Probanden im Alter von 30 bis 55 Jahren haben die in der DDR handelsübliche Haarkurpackung "Florena-Aminat", (Hersteller: VEB Hydrierwerk Rodleben, Packungsinhalt: 100 ml) mit einem Zusatz von 34 mg NaSCN je Anwendung zur Haarpflege eingesetzt. Hierzu wurde 1/4 des Packungsinhalts mit der etwa zweibis dreifachen Menge warmen Wassers verrührt und 8 ml einer wäßrigen NaSCN-Lösung (NaSCN-Gehalt 4,25 mg/ml) zugegeben. Vor Anwendung der Kurpackung wurden die Haare mit handelsüblichem Shampoo gewaschen, die Haare mit einem Handtuch abgetrocknet und auf das noch feuchte Haar die Kurpackung aufgetragen, mittels Durchkämmen verteilt, mit einer Plastekappe abgedeckt und nach 20 bis 60 min Einwirkungszeit die Packung mit warmem Wasser ausgewaschen. Die Haare wurden ein- bzw. zweimal pro Woche gewaschen, die Kurpackung wurde wöchentlich einmal angewendet. Bei allen Probanden wurden keine Haarfärbemittel angewendet.
Bereits nach der ersten Anwendung war das Haar weich, ließ sich leichter frisieren und hatte einen besseren Glanz. Die Erprobung verlief über einen Zeitraum von mehr als einem Jahr mit den gleichen günstigen Ergebnissen. Vor diesem Erprobungszeitraum wurde die selbe Haarpackung, jedoch ohne Thiocyanatzusatz angewendet. Subjektiv war auffällig, daß sich der Haarzustand (Glanz, Frisierbarkeit, Elastizität) nach dem Zusatz von NaSCN zur Kurpackung im Vergleich zum vorherigen Haarzustand deutlich verbessert hat.

Beispiel 5

In einem Modellversuch (Versuchstier: Maus) zur experimentellen Leukopoesehemmung, Behandlung durch intraperitoneale Injektion (in die Bauchhöhle) von Cyclophosphamid, 74 mg/kg Körpermasse/Tag, 7 Tage lang, wurden als klinische Nebenbefunde stark struppiges Fell (ab 3. Tag), Haarausfall (ab 5. Tag) und Diarrhoe bei 6 von 10 Tieren beobachtet. Erhielten die Tiere parallel dazu subcutan (durch Injektion unter die haut) 64 mg NaSCN/kg Körpermasse/Tag, ebenfalls 7 Tage lang, war der klinische Allgemeinzustand deutlich besser. Das Fell erschien unverändert, es war kein Haarausfall auffällig. Nur 2 von 10 Versuchstieren hatten Durchfall. Die Leukopoesehemmung war deutlich reduziert.

Beispiel 6

In der industriemäßigen Lämmermast (Merinofleischschaf, Fütterung mit Pelletkonzentrat) wurde ein Gemisch von KSCN und NaSCN (Mischungsverhältnis 3:2) zweimal wöchentlich in einer Einzeldosis von jeweils 12 mg $SCN^-$/kg Körpermasse oral mit dem Tränkwasser verabreicht. Diese Dosierung entspricht etwa der 4fachen Menge, wie sie bei Weidehaltung mit der Nahrung aufgenommen wird. Die erste Applikation wurde am Tag der Aufstallung nach Zusammenstellung der Versuchs- und Kontrollgruppen vorgenommen. Die weiteren Gaben erfolgten alternierend jeweils im Abstand von 3 bis 4 Tagen, insgesamt viermal. Am 10. Tag nach der Aufstallung wurden die Tiere geschoren.
In der erfindungsgemäß mit $SCN^-$ versorgten Gruppe war ein Mehrertrag an Schweißwolle um durchschnittlich 6 % nachweisbar (Tabelle 1).

Tabelle 1 Schweißwollertrag bei Lämmerintensivmast mit Schur am 10. Tag der Aufstallung

| Tiergruppe | n | KM (kg)/Tier ($\bar{x}$) | Wollertrag (g)/Tier ($\bar{x}$) |
|---|---|---|---|
| Versuchsgruppe | 99 | 26 | 1495 |
| Kontrollgruppe | 99 | 26 | 1415 |

KM - Körpermasse

n - Anzahl der untersuchten Tiere

Beispiel 7

Die Versuchsbedingungen entsprechen dem Beispiel 6, es wurde lediglich die Applikationsdauer auf 11 Gaben erhöht. In der erfindungsgemäß mit SCN⁻ versorgten Gruppe wurde bei der gewählten verlängerten Applikationsphase ein Mehrertrag an Schweißwolle um 9 % erreicht. Die Stapellänge wurde um 3,4 % erhöht (Tabelle 2).

Tabelle 2 Schweißwollertrag bei Lämmerintensivmast mit Schur am 34. Tag der Aufstallung

| Tiergruppe | n | KM (kg)/Tier ($\bar{x}$) | Wollertrag (g)/Tier ($\bar{x}$) | (s) | Stapellänge mm/Tier ($\bar{x}$) | (s) |
|---|---|---|---|---|---|---|
| Versuchs-gruppe | 98 | 23,0 | 933 [1] | 13 | 43,2 | 2,6 |
| Kontroll-gruppe | 98 | 22,7 | 850 | 25 | 41,8 | 3,6 |

KM - Körpermasse

n - Anzahl der untersuchten Tiere

[1] Unterschied zur Kontrollgruppe mit $\alpha \leq$ 5 % signifikant

Zusätzlich wurde die Wollqualität verbessert, ermittelt am Merkmal der Feinheit (Tabelle 3).

Tabelle 3 Feinheit der Wolle bei Lämmerintensivmast mit Schur am 34. Tag der Aufstallung

| | Feinheit x (in Mikrometer) | | | | | |
|---|---|---|---|---|---|---|
| Tiergruppe | Keule | | Flanke | | Schulterblatt | |
| | n | $(\bar{x})$ | n | $(\bar{x})$ | n | $(\bar{x})$ |
| Versuchsgruppe | | | | | | |
| Tag der Auf- | | | | | | |
| stallung | 47 | 22,9 | 61 | 22,6 | 41 | 22,2 |
| Tag der Schur | | | | | | |
| (34. Tag) | 47 | 23,9 | 61 | 23,4 | 41 | 23,2 |
| | | | | | | |
| Kontrollgruppe | | | | | | |
| Tag der Auf- | | | | | | |
| stallung | 74 | 24,3 | 55 | 22,3 | 56 | 24,3 |
| Tag der Schur | | | | | | |
| (34. Tag) | 74 | 24,1 | 55 | 22,3 | 56 | 23,0 |
| | | | | | | |
| Differenz | | | | | | |
| zwischen | | | | | | |
| Ausgangs- u. | | | | | | |
| Endwert (in %) | | | | | | |
| | | | | | | |
| Versuchsgruppe | | 4,5[1] | | 3,4[1] | | 4,7[1] |
| Kontrollgruppe | | − 0,5 | | −0,1 | | −0,6 |

[1] Unterschied zur Kontrollgruppe mit $\alpha < 5\%$ signifikant

n − Anzahl der untersuchten Tiere

Als Ausdruck einer besseren Gesamtentwicklung der Tiere war in der Versuchsgruppe eine größere Körpermassezunahme als in der Kontrollgruppe feststellbar (Tabelle 4).

Tabelle 4 Körpermassezunahme bei industriemäßiger Lämmermast

| Tiergruppe | Zunahme in Gramm/Tag (g/d) | |
|---|---|---|
| | $(\bar{x})$ | $(s)$ |
| Versuchsgruppe | 248,1 | 114,0 |
| Kontrollgruppe | 232,3 | 94,4 |

Biologisch bedeutungsvoll im Hinblick auf die Gesamteinordnung des SCN⁻-Effekts auf die Stimulierung des Wollertrags ist der höhere SCN⁻-Gehalt in der Wollfaser zum Zeitpunkt der Schur bei den Versuchstieren im Vergleich zu den Kontrolltieren (Tabelle 5).

Die Befunde sind auch insofern bemerkenswert, als mit zunehmender Feinheit der Wolle (Zunahme der Feinheit von Keule über Flanke zum Schulterblatt) der SCN⁻-Gehalt in der Versuchsgruppe höher ist. In der Kontrollgruppe sind dagegen keine Unterschiede auffällig.

Tabelle 5 Thiocyanatgehalt in Wollproben verschiedener Körperregionen bei Intensivmastlämmern

| Tiergruppe | SCN⁻-Gehalt (mg/kg Wolle) | | | |
|---|---|---|---|---|
| | Schulter-blatt | Flanke | Keule | gesamt |
| Versuchsgruppe | 18,6 | 16,8 | 15,3 | $17,3 \pm 1,1$ [1] |
| Kontrollgruppe | 13,0 | 13,3 | 13,1 | $13,2 \pm 0,1$ |

Anzahl der untersuchten Haare je Untersuchungsbereich:
n = 48 ... 78
[1] Unterschied zur Kontrollgruppe mit $\alpha < 5\%$ signifikant

## Beispiel 8

Die Versuchsbedingungen entsprechen dem Beispiel 7 mit einer Applikation von 11 Gaben.

Die Versuchs- und Kontrollgruppen wurden von Zwillingspaaren gebildet, wobei jeweils ein Zwilling der Versuchs- bzw. der Kontrollgruppe zugeordnet wurde. Die mit SCN⁻ versorgten Zwillinge erbrachten einen Mehrertrag an Schweißwolle von 18 %. Die Stapellänge erhöhte sich um 3 % (Tabelle 6).

Tabelle 6 Schweißwollertrag bei Intensivmastlämmern mit Schur am 34. Tag der Aufstallung bei Zwillingsvergleich

| Tiergruppe | n | KM (kg)/ Tier ($\bar{x}$) | Wollertrag (g)/ Tier | | Stapellänge (mm)/Tier | |
|---|---|---|---|---|---|---|
| | | | ($\bar{x}$) | (s) | ($\bar{x}$) | (s) |
| Versuchsgruppe | 9 | 20 | 889[1] | 13 | 45,8 | 5,0 |
| Kontrollgruppe | 9 | 20 | 756 | 22 | 43,2 | 4,0 |

[1] Unterschied zur Kontrollgruppe mit $\alpha < 5\ \%$ signifikant

n - Anzahl der untersuchten Tiere

KM - Körpermasse

Beispiel 9

Bei konventioneller individueller Schafhaltung (Kreuzungstiere Ostfriesisches Milchschaf/Merinofleischschaf) wurde während der Stallhaltungsperiode (Fütterung mit Gerstenkleie, Runkelschnitzel und Heu) der Einfluß einer SCN⁻-Applikation innerhalb von 3 Jahren untersucht (1985 - 1987). Während der Periode der Stallhaltung (50 Tage im März - April) wurden wöchentlich zweimal insgesamt 16 Einzelgaben zu je 12 mg SCN⁻/kg oral verabreicht. Die Wolleigenschaften wurden zu Versuchsbeginn und zur Schur am Jahresende ermittelt. Von Mai bis Ende November waren die Tiere auf der Weide und wurden dann erneut aufgestallt. Bis April 1986 (128 Tage) wurde SCN⁻ analog verabreicht (41 Einzelgaben). Bei der Schur im Januar 1987 wurde erneut bewertet. Die Schur wurde bei den Alttieren 13 Monate nach Beginn der zusätzlichen SCN⁻-Versorgung vorgenommen, bei den Lämmern 11 Monate nach der Geburt.

Bei den Kontrolltieren (Alttiere) blieb der Wollertrag in drei aufeinanderfolgenden Jahren nahezu konstant (Tabelle 7). Das ist als Ausdruck dafür zu werten, daß die Haltungs- und Fütterungsbedingungen während des dreijährigen Untersuchungszeitraums annähernd gleichbleibend waren. In der Versuchsgruppe war dagegen von Jahr zu Jahr eine Ertragssteigerung feststellbar, von 1986 zu 1985 um 8 %, von 1987 im Vergleich zu 1985 um 18 % (Tabelle 7).

Tabelle   7    Schweißwollwachstum  in  Gramm/Tag  bei
                konventioneller  Schafhaltung

| Tiergruppe | n | $(\bar{x})$ | (s) |
|---|---|---|---|
| Versuchsgruppe | | | |
| 1985[1] | 6 | 10,9 | 2,6 |
| 1986 | 10 | 11,8 | 2,3 |
| 1987 | 9 | 12,9 | 5,0 |
| Kontrollgruppe | | | |
| 1985 [1] | 8 | 11,8 | 2,1 |
| 1986 | 9 | 11,4 | 2,0 |
| 1987 | 9 | 11,5 | 2,4 |

[1] Ausgangswert  zu  Untersuchungsbeginn

Das Wollwachstum der 1986 geborenen Lämmer betrug in der Kontrollgruppe 3,9 ± 1,1 Gramm/Tag, in der Versuchsgruppe 4,5 ± 0,8 Gramm/Tag.
Damit betrug der Wollmehrertrag etwa 15 % (Tabelle 8). Die Versuchsgruppe erhielt eine indirekte zusätzliche $SCN^-$-Versorgung über die Muttertiere in utero (3 Monate) sowie über die Muttermilch (2 Monate).

Tabelle   8      Schweißwollertrag   bei   konventioneller   Schaf-
                 haltung  (Lämmer)

| Tiergruppe | n | Wollertrag (g)/Tier |
|---|---|---|
| Versuchsgruppe [1] | 8 | 4501 |
| Kontrollgruppe | 5 | 3910 |

n = Anzahl  der  untersuchten  Tiere
[1]   indirekte  zusätzliche  $SCN^-$-Versorgung  in  utero
      (3 Monate) bzw. über die Muttermilch (2 Monate)

Während sich die Stapellänge bei den Alttieren in den drei Jahren praktisch nicht zwischen Kontroll- und Versuchsgruppe unterschied, war bei den Lämmern die Tendenz einer größeren Stapellänge (Zunahme um 28 %) feststellbar (Tabelle 9). Zugleich war der Zuwachs an Körpermasse tendentiell größer (um 8 %).

11

Tabelle 9    Körpermassezunahme und Stapellänge bei
konventioneller Schafhaltung (Lämmer)

| Tiergruppe | n | Massezunahme in Gramm/Tag/Tier | | Stapellänge in mm | |
| --- | --- | --- | --- | --- | --- |
| | | ($\bar{x}$) | s | ($\bar{x}$) | s |
| Versuchsgruppe | 14 | 112,4 | 44,7 | 33 | 5 |
| Kontrollgruppe | 14 | 103,8 | 27,6 | 26 | 10 |

n - Anzahl der untersuchten Tiere

Es konnte festgestellt werden, daß bei einer zusätzlichen SCN⁻ -Versorgung der Muttertiere während der Hochträchtigkeit und der Lactation (Periode des Säugens) eine geringere Lämmersterblichkeit zu verzeichnen war (Tabelle 10).

Tabelle 10    Lämmeraufzuchtleistung

| | Mutter-tiere | Lebend-geborene Lämmer | Lämmer/ Mutter-tier | Lämmer-veren-dungen | Aufzucht-rate/Mut-tertier |
| --- | --- | --- | --- | --- | --- |
| | (n) | (n) | | (n) | |
| Versuchsgruppe | 8 | 14 | 1,8 | 3 | 1,4 |
| Kontrollgruppe | 9 | 14 | 1,6 | 4 | 1,1 |

Beispiel 10

Albinomeerschweinchen wurden mit Hilfe von Bariumsulfid auf dem Rücken nach vorheriger elektrischer Rasur depiliert und danach 2 Versuchs- und 1 Kontrollgruppe gebildet. Die Tiere von Versuchsgruppe T 1 wurden einmal täglich, sieben Wochen lang in einer NaSCN-Lösung für 10 min gebadet (300 mg NaSCN/l Wasser, Wassertemperatur 37 °C). Die Tiere der Versuchsgruppe T 2 erhielten während desselben Zeitraums täglich oral per Pipette 32 mg NaSCN/kg Körpermasse. Bis 10 Tage vor Applikationsende wurde die chemische Depilation im Abstand von 2 bis 3 Tagen wiederholt. Damit sollte der SCN⁻ -Einfluß vorrangig am wachsenden Haar untersucht werden. Am Ende des siebenwöchigen Versuchszeitraumes wurde bei Versuchs- und Kontrolltieren die Wachstumsgeschwindigkeit der Haare innerhalb der letzten 10 Tage nach der letzten Depilation ermittelt. 4 Tage später wurde bei den Tieren der Versuchs- und der Kontrollgruppe ein Trichogramm nach Orfanos (Orfanos, C. E.: Haar und Haarkrankheiten. F: scher-Verlag Stuttgart/New York, 1979) mit folgenden Merkmalen erstellt:
   - Durchschnittliche Haardicke in Mikrometer einer Haarwachstumsphase (Tabelle 11),

- Anteile der Zyklusphasen innerhalb der Versuchsgruppen (Tabelle 12),
- Wachstumsgeschwindigkeit der Haare (Tabelle 13),
- Haardichte (Tabelle 14).

Zusätzlich wurde der $SCN^-$-Gehalt in Haaren, Haut, Nebenniere, Milz, Schilddrüse, Leber und Muskel bestimmt.

In der Anagenphase, der Wachstumsphase der Haare, ist die Haardicke in beiden Versuchsgruppen im Vergleich zur Kontrollgruppe vermindert (Tabelle 11).

Tabelle 11    Durchschnittliche Haardicke in verschiedenen Haarwachstumsphasen bei Albinomeerschweinchen

|  |  |  | Parameter | | |
|---|---|---|---|---|---|
| Tiergruppe |  |  | Haardicke in Mikrometer | | |
| Haarstadium | z | n | $(\bar{x})$ | s | % |
| Kontroll- | 17 |  |  |  |  |
| gruppe   Anagenhaare |  | 740 | 37,9 | 14,9 | 100 |
| dysplastische |  |  |  |  |  |
| Anagenhaare |  | 56 | 28,8 | 14,5 | 100 |
| Katagenhaare |  | 296 | 51,9 | 17,2 | 100 |
| Telogenhaare |  | 577 | 85,2 | 24,1 | 100 |
| Anwendung von NaSCN |  |  |  |  |  |
| als Bad (Gruppe T 1) | 15 |  |  |  |  |
| Anagenhaare |  | 875 | 30,9 | 11,8 | 81 |
| dysplastische |  |  |  |  |  |
| Anagenhaare |  | 23 | 29,0 | 9,9 | 103 |
| Katagenhaare |  | 154 | 60 | 19,4 | 116 |
| Telogenhaare |  | 349 | 86,5 | 23,4 | 101 |
| orale Gabe von NaSCN | 16 |  |  |  |  |
| (Gruppe T 2) |  |  |  |  |  |
| Anagenhaare |  | 840 | 34,3 | 11,3 | 90 |
| dysplastische | 36 | 35,9 | 15 | 124 | |
| Anagenhaare |  |  |  |  |  |
| Katagenhaare |  | 157 | 55,6 | 14 | 107 |
| Telogenhaare |  | 519 | 83,7 | 22,8 | 98 |

z - Anzahl der untersuchten Tiere

n - Anzahl der untersuchten Einzelhaare

 

Unter dem Einfluß von SCN⁻ kommt es zu einer signifikanten Steigerung der in der anagenen Phase, also der Wachstumsphase der Haare, befindlichen Haaranteile. Demgegenüber nimmt der Anteil der in der Ruhephase (telogenen Phase) befindlichen Haare ab. Die anderen Wachstumsphasen sind zu Gunsten der anagenen Phase relativ vermindert (Tabelle 12).

Tabelle   12     Anteile der Zyklusphasen innerhalb der Tiergruppen
                 bei Albinomeerschweinchen

| Versuchsgruppe | Anagen-haare | Dysplast. Anagenhaare | Katagen-haare | Telogen-haare |
|---|---|---|---|---|
| Untersuchte Haare der Kontrollgruppe (Vergleichsgruppe) | 740 | 56 | 296 | 577 |
| n = 1669 (17 Tiere) | 44 % | 3 % | 18 % | 35 % |
| Anwendung von NaSCN als Bad (Versuchsgruppe T 1) | 875 | 23 | 154 | 349 |
| untersuchte Haare n = 1401 (15 Tiere) | 63 % [1] | 2 % | 10 % | 25 % |
| orale Anwendung von NaSCN (Versuchsgruppe T 2) | 840 | 36 | 157 | 519 |
| untersuchte Haare n = 1552 (16 Tiere) | 55 % | 3 % | 7 % [1] | 35 % |

[1] Unterschied zur Kontrollgruppe mit $\alpha < 5\,\%$ signifikant

Im Vergleich zur Kontrollgruppe betrug der Zuwachs der in der Anagenphase befindlichen Haare bei Anwendung von NaSCN als Bad 41 %, bei oraler Anwendung von NaSCN 22 %. 10 Tage nach Rasur und Depilation ist die Wachstumsgeschwindigkeit bei den Versuchsgruppen um 5 % erhöht (Tabelle 13).

Tabelle 13    Wachstumsgeschwindigkeit als Ausdruck der Haar-
              längenzunahme in 10 Tagen bei Albino-
              meerschweinchen

| Versuchsgruppe | Mittlerer Haarlängen- zuwachs in 10 Tagen (Angaben in mm) | | |
|---|---|---|---|
| | $(\bar{x})$ | % | s |
| Untersuchte Haare der Kontrollgruppe (Vergleichsgruppe) n = 85 (17 Tiere) | 7,0 | 100 | 0,75 |
| Anwendung von NaSCN als Bad (Versuchsgruppe T 1) untersuchte Haare n = 90 (18 Tiere) | 7,4 | 105,6 | 0,6 |
| orale Anwendung von NaSCN (Versuchsgruppe T 2) untersuchte Haare n = 90 (18 Tiere) | 7,4 | 105,4 | 0,68 |

Die Haardichte war in den Versuchsgruppen ebenfalls erhöht (Tabelle 14).

Tabelle 14. Haardichte bei Albinomeerschweinchen

| Versuchsgruppe | Anzahl Haare/$cm^2$ | | |
|---|---|---|---|
| | $\bar{x}$ | % | s |
| Untersuchte Gesichtsfelder (je $cm^2$) Kontrollgruppe (Vergleichsgruppe) n = 85 (17 Tiere) | 49,2 | 8,9 | 100 |
| Untersuchte Gesichtsfelder (je $cm^2$) Anwendung von NaSCN als Bad (Versuchsgruppe T 1) n = 75 (15 Tiere) | 53,3 | 10,4 | 108,4 |
| Untersuchte Gesichtsfelder (je $cm^2$) — orale Gabe von NaSCN (Versuchsgruppe T 2) n = 86 (16 Tiere) | 52,0 | 12,6 | 105,7 |

In beiden Versuchsgruppen war der $SCN^-$-Gehalt in den Haaren deutlich erhöht. Hierbei ist in Rechnung zu stellen, daß die Tiere vor der Bestimmung des $SCN^-$-Gehaltes nach dem Bad dreimal intensiv unter fließendem Leitungswasser abgespült wurden und anschließend jeweils zweimal in warmes Leitungswasser eingetaucht wurden, um zu erreichen, daß so wenig wie möglich $SCN^-$ direkt auf den Haaren verbleibt. Da auch bei der oralen Applikation von $SCN^-$ sich der $SCN^-$-Gehalt in den Haaren um ein Vielfaches erhöht, sprechen die Ergebnisse dafür, daß es zu einer aktiven Anreicherung von $SCN^-$ in den Haaren beim Haarwachstum kommt, was in Verbindung mit den beobachteten fördernden Einflüssen auf das Haarwachstum nicht überrascht.

Bei Anwendung von NaSCN als Bad kam es erwartungsgemäß zu keinen Veränderungen des $SCN^-$-Gehalts in Nebenniere, Milz, Schilddrüse und Muskel. In der Haut war die $SCN^-$-Konzentration wiederum erhöht.

Beispiel 11

Im Unterschied zum siebenwöchigen Applikationszeitraum in den Monaten Februar/März im Beispiel 10 wurde im Beispiel 11 im Wiederholungsversuch der Applikationszeitraum bei gleicher Dosierung und Gruppenbildung auf zehn Wochen ausgedehnt und die Versuche in den Monaten September bis November durchgeführt.

Unter dem Einfluß von SCN⁻ kommt es wiederum zu einer signifikanten Zunahme der sich in der anagenen Phase befindlichen Haare (Tabelle 15). Im Vergleich zum Beispiel 9 ist auffällig, daß bei einer größeren Anzahl ruhender Haarfolikel, wie sie im Herbst feststellbar war, der stimulierende SCN⁻-Effekt stärker zur Ausprägung kommt. Wiederum ist bei Anwendung als Bad der Einfluß größer als bei oraler Anwendung.

Tabelle 15  Anteile der Zyklusphasen innerhalb der Tiergruppen bei Albinomeerschweinchen

| Versuchs-gruppe | Anagen-haare | Dysplast. Anagen-haare | Katagen-haare | Telogen-haare | dystrophe Haare |
|---|---|---|---|---|---|
| Untersuchte Haare der Kontrollgruppe (Vergleichsgruppe) n = 1000 (10 Tiere) | 194 19 % | 28 3 % | 530 53 % | 199 20 % | 49 5 % |
| Anwendung von NaSCN als Bad n = 2400 (24 Tiere) | 1387 58 %[1;3] | 74 3 | 686 29 | 158 6 %[2] | 96 4 % |
| orale Anwendung von NaSCN n = 2800 (28 Tiere) | 1338 48 %[1] | 25 1 % | 854 31 % | 546 19 % | 36 1 % |

n - Anzahl der untersuchten Einzelhaare

[1] Unterschied zur Kontrollgruppe mit $\alpha < 0,2\ \%$ signifikant

[2] Unterschied zur Kontrollgruppe mit $\alpha < 5\ \%$ signifikant

[3] Unterschied zur Gruppe mit oraler Anwendung

$\alpha < 5\ \%$ signifikant

10 Tage nach Rasur und Depilation ist die Haarlängenzunahme in den Versuchsgruppen um 7 % bzw. 14 % höher als in der Kontrollgruppe (Tabelle 16). Der Effekt war wiederum deutlicher ausgeprägt als in Beispiel 10. Offenbar sind jahreszeitliche Einflüsse von Bedeutung.

Tabelle 16     Wachstumsgeschwindigkeit als Ausdruck der Haarlängenzunahme innerhalb von 10 Tagen bei Albinomeerschweinchen

| Versuchsgruppe | Mittlerer Haarlängen-zuwachs in 10 Tagen (Angaben in mm) $(\bar{x})$ | % | s |
|---|---|---|---|
| Untersuchte Haare der Kontrollgruppe n = 100 (10 Tiere) | 7,2 | 0,7 | 100 |
| Anwendung von NaSCN als Bad, untersuchte Haare n = 250 (25 Tiere) | 7,7[1] | 0,8 | 107 |
| orale Anwendung von NaSCN, untersuchte Haare n = 280 (28 Tiere) | 8,1[1] | 0,8 | 114 |

[1] Unterschied zur Kontrollgruppe mit $\alpha < 5$ % signifikant

Auch die Haardichte hatte wie in der ersten Versuchsserie bei Thiocyanatapplikation zugenommen (Tabelle 17), wobei das Ausmaß der biologischen Effektivität wiederum in der Herbstserie deutlich höher war.

Tabelle 17 Haardichte bei Albinomeerschweinchen

| Versuchsgruppe | Anzahl Haare/cm$^2$ | | |
|---|---|---|---|
| | $(\bar{x})$ | s | % |
| Untersuchte Gesichtsfelder (je 1 cm$^2$) Kontrollgruppe n = 50 (10 Tage) | 38,4 | 4,8 | 100 |
| Untersuchte Gesichtsfelder (je 1 cm$^2$) Anwendung von NaSCN als Bad n = 125 (25 Tiere) | 53,0 [1] | 8,9 | 138 |
| Untersuchte Gesichtsfelder (je 1 cm$^2$) orale Gabe von NaSCN n = 140 (28 Tiere) | 51,1 [2] | 7,8 | 133 |

[1] Unterschied zur Kontrollgruppe mit $\alpha < 0,2$ % signifikant
[2] Unterschied zur Kontrollgruppe mit $\alpha < 5$ % signifikant

Die Befunde am Versuchstier Meerschweinchen sind insofern besonders bedeutungsvoll, als auf Grund der Mosaikmauser dieser Species die Haarbildung der des Menchen sehr ähnlich ist.

Beispiel 12

Industriemäßig gehaltenen weiblichen und männlichen Standardnerzen (Mustela vision), durchschnittliches Alter 8 Wochen, durchschnittliche Körpermasse zu Versuchsbeginn 730 g (Rüden) bzw. 560 g (Fähen), wurde ab 3. Lebensmonat mit dem Futtergemisch, das aus unterschiedlichen Komponenten - hauptsächlich aus Milchpulver, Innereien von Rind und Schwein sowie Fisch bestand - täglich KSCN nach folgendem Applikationsschema verabreicht (vgl. Tabelle 18).

Tabelle 18 Alimentär aufgenommene Thiocyanatmengen beim Nerz

| Tiergruppe | SCN⁻-Aufnahme in mg SCN⁻/kg Körpermasse im | | | | | |
|---|---|---|---|---|---|---|
| | 3. Lebensmonat[1] | | 4. Lebensmonat[1] | | 7. Lebensmonat[2] | |
| | Rüden | Fähen | Rüden | Fähen | Rüden | Fähen |
| Kontroll- gruppe | 0,7 | 0,9 | 0,5 | 0,7 | 0,2 | 0,4 |
| zusätzli- che orale KSCN-Gabe | 2,0 | 2,6 | 0,9 | 1,2 | 0,7 | 1,3 |

KM – Körpermasse
[1] SCN⁻-Gehaltsbestimmung im Futter am 1. Tag des Monats
[2] SCN⁻-Gehaltsbestimmung im Futter am letzten Tag des 7. Lebensmonats

Die Lebendbewertung der Tiere gemäß DDR-Standard TGL 24300/02 erfolgte nach den Merkmalen Fellfarbe und Fellstruktur. In der erfindungsgemäß mit KSCN versorgten Tiergruppe war eine um 6,9 % bessere Fellstruktur nachweisbar (Tabelle 19).

Tabelle 19 Lebendbewertung der Standardnerze am 147. Versuchstag

| Tiergruppe | n | Punktzahl Fellfarbe | Punktzahl Fellstruktur |
|---|---|---|---|
| Versuchsgruppe | 99 | 2,13 | 1,87 |
| Kontrollgruppe | 97 | 2,13 | 1,75 |

n – Anzahl der untersuchten Tiere

Die bessere Fellstruktur der mit KSCN behandelten Nerze wurde durch die Ergebnisse der Fellbewertung der bearbeiteten Felle bestätigt (Tabelle 20).

Die Begutachtung erfolgte dabei nach folgenden Kriterien:

1. Unterscheiden sich die Felle der Kontroll- und Versuchsgruppe in der Länge der Grannenhaare?

Die Bewertung wurde wie folgt vorgenommen:
- 3 Punkte = mittlere Granne, gutes Verhältnis zur Länge der Unterwolle,
- 2 Punkte = etwas zu lange bzw. etwas zu kurze Granne im Verhältnis zur Länge der Unterwolle,
- 1 Punkt = sehr lange bzw. sehr kurze Granne im Verhältnis zur Unterwolle.

2. Unterscheiden sich die Felle der Kontroll- und Versuchsgruppe in der Dichte sowie in der Gleichmäßigkeit der Begrannung?

Hierbei wurde die Einstufung nach folgenden Kriterien vorgenommen:
- 2 Punkte = mittel,
- 1 Punkt = schlecht.

3. Unterscheiden sich die Felle der Kontroll- und Versuchsgruppe in der Haarstärke und -elastizität?

Die vorgegebenen Punkte bedeuten:
- 3 Punkte = ideal,
- 2 Punkte = mittel,
- 1 Punkt = schlecht.

Es wurde festgestellt, daß die Grannenhaare bei den Versuchstieren im Verhältnis zur Unterwolle geringfügig länger werden als die der Kontrolltiere. Die Dichte und Gleichmäßigkeit der Begrannung ist bei den Versuchstieren um 5,6 %, die Elastizität und Stärke des Haares um 4,8 % besser als bei den Kontrolltieren (Tabelle 20).

Tabelle 20    Bewertung der Felle der Standardnerze nach der Bearbeitung

|  |  |  | Punktzahl |  |
| --- | --- | --- | --- | --- |
| Tiergruppe | n | Länge der Grannen- haare | Dichte u. Gleich- mäßigkeit der Be- grannung | Haarstärke und -elasti- zität |
| Versuchs- gruppe | 95 | 2,55 | 2,26 | 2,61 |
| Kontroll- gruppe | 97 | 2,58 | 2,14 | 2,49 |

n - Anzahl der untersuchten Einzelhaare

Ferner wurde in der Versuchsgruppe eine bessere Körpermassezunahme (Tabelle 21) und als Folge dessen eine größere Körper- und Fellänge (Tabelle 22) als in der Kontrollgruppe erreicht.

Tabelle 21    Körpermassezunahme der Standardnerze nach 148 Versuchstagen

| Tier- gruppe | n | Einstallungs- lebendmasse E | Körpermasse (g) Ausstallungs- lebendmasse A | Differenz A-E |
|---|---|---|---|---|
| Versuchs- gruppe | 99 | 668 | 1813 | 1145 |
| Kontroll- gruppe | 97 | 622 | 1759 | 1137 |

n - Anzahl der untersuchten Tiere

Tabelle 22    Körperlänge und Fellänge der Standardnerze nach Bearbeitung

| Tiergruppe | n | Körperlänge (cm) | Fellänge nach Bear- beitung (cm) |
|---|---|---|---|
| Versuchs- gruppe | 77 | 44 | 66 |
| Kontroll- gruppe | 97 | 42 | 65 |

n - Anzahl der untersuchten Tiere

Im Hinblick auf die physiologische Wirksamkeit von SCN⁻ auf die Struktureigenschaften des Haarkleides beim Nerz ist von Bedeutung, daß der SCN⁻-Serumspiegel der Versuchstiere im Versuchszeitraum mit 6 - 8 mg SCN⁻/l deutlich höher als der der Kontrolltiere (4 - 5 mg SCN⁻/l) war.

Beispiel 13

Im Unterschied zum Beispiel 10 wurde der Applikationszeitraum von NaSCN auf 4 Wochen verkürzt (Februar/März) und NaSCN nur als Bad einmal täglich angewendet. Die Konzentration im Badewasser betrug, bezogen auf den Anteil von SCN-Ionen, 0,5 bzw. 1 %. Die Tiere wurden nicht 10 min gebadet, sondern unter manuellen Waschbewegungen im Badewasser intensiv bis auf die Haut benetzt und feucht in die Käfige zu-

23

rückgesetzt. Nach etwa 14 min war das Fell getrocknet, d. h. die Einwirkungszeit von SCN⁻ in der feuchten Phase umfaßte jeweils etwa diesen Zeitraum. Die Kontrolltiere wurden unter analogen Bedingungen in 37 °C warmem Leitungswasser gebadet. Zu Versuchsbeginn wurden die Tiere zuerst elektrisch vorrasiert und dann mit Bariumsulfid chemisch depiliert. 13 Tage vor Versuchsende wurden alle Tiere erneut chemisch depiliert. Jede Gruppe bestand aus 8 Versuchstieren.

Durch beide SCN⁻-Konzentrationen wird eine signifikante Steigerung der in der Anagenphase befindlichen Haaranteile bewirkt (Tab. 23). Dieser günstige Effekt wird noch dadurch unterstrichen, daß der Anteil der in der Telogen- und in der Katagenphase befindlichen Haare abnimmt. Auch der Anteil dysplastischer Anagenhaare und dystropher Haare ist in der Tendenz reduziert. Damit wird auch durch diese SCN⁻-Dosierung eine ausgeprägte Förderung der Haarbildung mit Verschiebung der Haarzyklusphasen zugunsten der anagenen Wachstumsphase induziert.

Tabelle 23  Anteile der Zyklusphasen innerhalb der Tiergruppen bei Albinomeerschweinchen

| Versuchsgruppe | Anagen-haare | dysplasti-sche Ana-genhaare | Katagen-haare | Telogen-haare | dystro-phe Haare |
|---|---|---|---|---|---|
| Untersuchte Haare der Kontrollgruppe | 285 | 22 | 122 | 360 | 10 |
| n = 800 (8 Tiere) | 36 % | 3 % | 15 % | 45 % | 1 % |
| Anwendung von NaSCN als Bad (5 g SCN⁻/1) | 423 [1] | 8 | 22 [2] | 238 [2] | 9 |
| n = 700 (7 Tiere) | 61 % | 1 % | 3 % | 34 % | 1 % |
| Anwendung von NaSCN als Bad (10 g SCN⁻/1) | 431 [1] | 8 | 55 [2] | 300 [2] | 6 |
| n = 800 (8 Tiere) | 54 % | 1 % | 7 % | 37 % | 1 % |

[1] Unterschied zur Kontrollgruppe mit $\alpha < 1$ % signifikant
[2] Unterschied zur Kontrollgruppe mit $\alpha < 5$ % signifikant

Trotz des nur 4-wöchigen Applikationszeitraumes wird die Wachstumsgeschwindigkeit gefördert, wenn auch erwartungsgemäß nicht in dem Ausmaß wie bei der 7- bzw. 10-wöchigen Anwendungsdauer im Beispiel 10 und 11 (Tab. 24).

Tabelle 24    Wachstumsgeschwindigkeit als Ausdruck der Haar-
              längenzunahme innerhalb von 13 Tagen bei Albino-
              meerschweinchen

----------------------------------------------------------------

Versuchsgruppe                          Haarlänge (mm)

                                        $\bar{x}$    s    %

----------------------------------------------------------------

Kontrollgruppe,
untersuchte Haare                       9,1   1,0   100
n = 80 (8 Tiere)

----------------------------------------------------------------

Anwendung von NaSCN als Bad
(5 g SCN$^-$/1),
untersuchte Haare
n = 70 (7 Tiere)                        9,3   0,9   102

----------------------------------------------------------------

Anwendung von NaSCN als Bad
(10 g SCN$^-$/1),
untersuchte Haare
n = 80 (8 Tiere)                        9,4   1,0   103   .

----------------------------------------------------------------

## Patentansprüche

1. Mittel zur qualitativen und quantitativen Förderung des Haarwuchses bei Mensch und Nutztier, dadurch gekennzeichnet, daß es neben üblichen Hilfs- und Trägerstoffen als einzigen wirksamen Bestandteil ein Salz oder Salze der Thiocyansäure enthält.

2. Mittel nach Anspruch 1, dadurch gekennzeichnet, daß es Natrium- und bzw. oder Kaliumthiocyanat enthält.

3. Mittel nach Anspruch 2 dadurch gekennzeichnet, daß es ein Gemisch aus Kaliumthiocyanat und Natriumthiocyanat enthält, bei dem in Abhängigkeit von der Anwendung der Anteil von Kaliumthiocyanat oder Natriumthiocyanat überwiegt.

4. Mittel nach Anspruch 1, dadurch gekennzeichnet, daß es ein Salz oder Salze der Thiocyansäure in einer Konzentration von bis zu 1 Gewichtsprozent Thiocyanationen zur Anwendung beim Nutztier bzw. bis zu 0,1 Gewichtsprozent Thiocyanationen zur Anwendung beim Menschen enthält.

5. Verfahren zur Verbesserung des Wachstums und der Qualität von Haar, Wolle, Pelz, Federn oder Leder in der Tierhaltung sowie zur Pflege der Haare und zur Erhaltung bzw. Förderung des Haarwuchses beim Menschen durch Verabreichung einer wirksamen Menge eines Salzes oder von Salzen der Thiocyansäure als alleiniger Wirkstoff neben üblichen Hilfs- und Trägerstoffen.

**6.** Verfahren nach Anspruch 5, dadurch gekennzeichnet, daß die Verabreichung extern (topisch) durch Zugabe zum Badewasser, zu Haarwässern, Shampoos, Kosmetika oder Pflegemitteln, oder durch Applikation vegetabiler thiocyanathaltiger Rohstoffe und/oder innerlich (oral bzw. alimentär) erfolgt.

**7.** Verfahren nach Anspruch 5 und 6, dadurch gekennzeichnet, daß die Salze der Thiocyansäure in einem Dosisbereich von 0,001% bis 0,1% beim Menschen bzw. von 0,001% bis 1,0% beim Nutztier in gelöster Form extern (topisch) angewendet werden.

**8.** Verfahren nach Anspruch 5 und 6, dadurch gekennzeichnet, daß die Salze der Thiocyansäure in einem Dosisbereich von 0,2 bis 1 mg/kg Körpermasse/Tag beim Menschen bzw. in einem Dosisbereich von 0,2 bis 70 mg/kg Körpermasse/Tag beim Nutztier verabreicht werden.

**9.** Verfahren nach einem der Ansprüche 5 bis 7, dadurch gekennzeichnet, daß als Wirkstoff Natrium- und bzw. oder Kaliumthiocyanat eingesetzt wird.

**10.** Verfahren nach Anspruch 9, dadurch gekennzeichnet, daß ein Gemisch aus Natrium- und Kaliumthiocyanat eingesetzt wird, bei dem in Abhängigkeit von der Anwendung der Anteil von Natriumthiocyanat oder Kaliumthiocyanat überwiegt, wobei für die Anwendung beim Menschen vorzugsweise der Anteil von Natriumthiocyanat dominiert.

## Claims

**1.** An agent for qualitatively and quantitatively supporting growth of the hair in man and in an useful animal, characterized in that it contains a salt or salts of thiocyanic acid as the only active ingredient in addition to conventional auxiliary and carrier materials.

**2.** The agent according to claim 1, characterized in that it contains sodium thiocyanate and/or potassium thiocyanate.

**3.** The agent according to claim 2, characterized in that it contains a mixture of potassium thiocyanate and sodium thiocyanate, wherein, dependently on the application thereof, the proportion of potassium thiocyanate or of sodium thiocyanate is predominant.

**4.** The agent according to claim 1, characterized in that it contains a salt or salts of thiocyanic acid in a concentration of up to 1% by weight of thiocyanate ions for the application to a useful animal or of up to 0.1% by weight of thiocyanate ions for the application to man.

**5.** A process for improving the growth and the quality of hair, wool, fur, feathers or leather in keeping animals and for hair care and for maintaining and supporting growth of the hair in man by administering an effective amount of a salt or of salts of thiocyanic acid as the only active ingredient in addition to conventional auxiliary and carrier materials.

**6.** The process according to claim 5, characterized in that the administration is effected externally (topie ally) by the addition to bath water, to hair tonics, shampoos, cosmetics or care treatment agents, or by the application of vegetable thiocyanate-containing raw materials and/or internally (orally or alimentarily).

**7.** The process according to claims 5 and 6, characterized in that the salts of the thiocyanic acid are externally (topically) administered in a dosage range of from 0.001% to 0.1% to man and of 0.001% to 1.0% to a useful animal in the dissolved state.

**8.** The process according to claims 5 and 6, characterized in that the salts of the thiocyanic acid are administered in a dosage range of from 0.2 to 1 mg per 1 kg of body mass per day to man and in a dosage range of from 0.2 to 70 mg per 1 kg of body mass per day to a useful animal.

**9.** The process according to anyone of claims 5 to 7, characterized in that sodium thiocyanate and/or potassium thiocyanate is/are employed as the active ingredient.

**10.** The process according to claim 9, characterized in that a mixture of sodium thiocyanate and potassium thiocyanate is employed, wherein, dependently on the application thereof, the proportion of sodium thiocyanate or of potassium thiocyanate is predominant, the proportion of sodium thiocyanate preferably dominating in the case of the application to man.

**Revendications**

1.  Produit pour la stimulation qualitative et quantitative de la pousse des cheveux/poils chez l'homme et l'animal utile, caractérisé en ce que, en plus d'adjuvants et véhicules usuels, il contient, en tant que seul composant actif, un sel ou des sels de l'acide thiocyanique.

2.  Produit selon la revendication 1, caractérisé en ce qu'il contient du thiocyanate de sodium et/ou du thiocyanate de potassium.

3.  Produit selon la revendication 2, caractérisé en ce qu'il contient un mélange de thiocyanate de potassium et de thiocyanate de sodium, dans lequel, en fonction de l'utilisation, la fraction de thiocyanate de potassium ou de thiocyanate de sodium prédomine.

4.  Produit selon la revendication 1, caractérisé en ce qu'il contient un sel ou des sels de l'acide thiocyanique à une concentration allant jusqu'à 1 % en poids d'ions thiocyanate pour l'utilisation chez l'animal utile ou jusqu'à 0,1 % en poids d'ions thiocyanate pour l'utilisation chez l'homme.

5.  Procédé pour l'amélioration de la pousse et de la qualité des poils, de la laine, de la fourrure, de plumes ou du cuir dans l'élevage ainsi que pour le soin des cheveux et pour le maintien ou la stimulation de la pousse des cheveux chez l'homme, par administration d'une quantité efficace d'un sel ou de sels de l'acide thiocyanique, en tant que seule substance active, en plus d'adjuvants et véhicules usuels.

6.  Procédé selon la revendication 5, caractérisé en ce l'administration est effectuée par voie externe (topique), par addition à l'eau de bain, à des lotions capillaires, shampooings, cosmétiques ou produits de soins, ou par application de matières premières végétales contenant des thiocyanates et/ou par voie interne (orale ou alimentaire).

7.  Procédé selon la revendication 5 ou 6, caractérisé en ce que les sels de l'acide thiocyanique sont utilisés par voie externe (topique), sous forme dissoute, dans un éventail de doses allant de 0,001 % à 0,1 % chez l'homme ou de 0,001 % à 1,0 % chez l'animal utile.

8.  Procédé selon la revendication 5 ou 6, caractérisé en ce que les sels de l'acide thiocyanique sont administrés dans un éventail de doses allant de 0,2 à 1 mg/kg de masse corporelle par jour chez l'homme, ou dans un éventail de doses allant de 0,2 à 70 mg/kg de masse corporelle par jour chez l'animal utile.

9.  Procédé selon l'une des revendications 5 à 7, caractérisé en ce que l'on utilise, en tant que substance active, le thiocyanate de sodium et/ou le thiocyanate de potassium.

10. Procédé selon la revendication 9, caractérisé en ce que l'on utilise un mélange de thiocyanate de sodium et de thiocyanate de potassium dans lequel, en fonction de l'utilisation, la fraction de thiocyanate de sodium ou de thiocyanate de potassium prédomine, la fraction de thiocyanate de sodium dominant de préférence pour l'utilisation chez l'homme.